# FASCICULE DE BREVET EUROPEEN

(11) **EP 2 323 738 B1**
(45) Date de publication et mention de la délivrance du brevet: **16.09.2015**
(21) Numéro de dépôt: 09784327.0
(22) Date de dépôt: 31.07.2009
(51) Int. Cl.: A61P 35/02, A61K 39/00, A61K 45/06, A61K 38/17

(54) **UTILISATION D'UNE FORME SOLUBLE D'HLA-G DANS LE TRAITEMENT DES HEMOPATHIES MALIGNES DE LA CELLULE B**
VERWENDUNG EINER LÖSLICHEN FORM VON HLA-G BEI DER BEHANDLUNG VON ANOMALER B-LYMPHOZYTEN-PROLIFERATION
USE OF A SOLUBLE FORM OF HLA-G IN THE TREATMENT OF ABNORMAL B-LYMPHOCYTE PROLIFERATION

(30) Priorité: 01.08.2008 FR 0804404
(43) Date de publication de la demande: 25.05.2011
(73) Titulaire: Commissariat à l'Énergie Atomique et aux Énergies Alternatives, 75015 Paris (FR)
(72) Inventeur: ROUAS-FREISS, Nathalie, F-75010 Paris (FR); CAROSELLA, Edgardo Delfino, F-75016 Paris (FR)
(74) Mandataire: Rançon, Xavier Lucien Abel
(86) Numéro de dépôt international: PCT/FR2009/000965
(87) Numéro de publication internationale: WO 2010/012912

(56) Documents cités:
- WO-A-2007/131575
- WO-A2-2005/002617
- FR-A- 2 810 047
- FR-A- 2 828 403
- MENIER C ET AL: "HLA-G5 turns off signalling by erythropoietin receptor" TISSUE ANTIGENS, MUNKSGAARD, COPENHAGEN, DK, vol. 68, no. 4, 1 octobre 2006 (2006-10-01), page 352, XP002450720 ISSN: 0001-2815
- REBMANN ET AL: "HLA-G in B-chronic lymphocytic leukaemia: Clinical relevance and functional implications" SEMINARS IN CANCER BIOLOGY, SAUNDERS SCIENTIFIC PUBLICATIONS, PHILADELPHIA, PA, US LNKD- DOI:10.1016/J.SEMCANCER.2007.06.011, vol. 17, no. 6, 9 novembre 2007 (2007-11-09), pages 430-435, XP022338889 ISSN: 1044-579X
- GROS F, SEBTI Y, DE GUIBERT S, BRANGER B, BERNARD M, FAUCHET R, AMIOT L: "Soluble HLA-G molecules are increased during acute leukemia, especially in subtypes affecting monocytic and lymphoid lineages" NEOPLASIA, vol. 8, no. 3, 1 mars 2006 (2006-03-01), pages 223-230, XP002585443

## Description

La présente invention est relative à une nouvelle utilisation des formes solubles d'HLA-G, dans le traitement des proliférations anormales des cellules B telles que les cancers liquides de type B.

Les antigènes du complexe majeur d'histocompatibilité (CMH), se divisent en plusieurs classes, les antigènes de classe I (HLA-A, HLA-B et HLA-C) qui présentent 3 domaines globulaires (α1, α2 et α3), et dont le domaine α3 est associé à la β2 microglobuline, les antigènes de classe II (HLA-DP, HLA-DQ et HLA-DR) et les antigènes de classe III (complément).

Les antigènes de classe I comprennent, outre les antigènes précités, d'autres antigènes, dits antigènes de classe I non classiques, et notamment les antigènes HLA-E, HLA-F et HLA-G.

La séquence du gène HLA-G (gène HLA-6.0) a été décrite par GERAGHTY et al., (Proc. Natl. Acad. Sci. USA, 1987, 84, 9145-9149) : il comprend 4396 paires de bases et présente une organisation intron/exon homologue à celle des gènes HLA-A, -B et -C. Ce gène comprend 8 exons, 7 introns et une extrémité non traduite 3'.

Le gène HLA-G diffère des autres gènes de classe I, en ce que le codon de terminaison de traduction, en phase, est localisé au niveau du deuxième codon de l'exon 6 ; en conséquence, la région cytoplasmique de la protéine codée par ce gène HLA-6.0 est plus courte que celle des régions cytoplasmiques des protéines HLA-A, -B et -C.

L'expression de ces isoformes est restreinte à quelques tissus tels que le trophoblaste (Kovats *et al.,* 1990), le thymus (Crisa *et al.,* 1997) et le pancréas (Cirulli *et al.,* 2006) dans des conditions non pathologiques.

D'autres recherches concernant cet antigène non classique de classe I (ISHITANI et al., Proc. Natl. Acad. Sci. USA, 1992, 89, 3947-3951) ont montré que le transcrit primaire du gène HLA-G peut être épissé de plusieurs manières et produit au moins 3 ARNm matures distincts : le transcrit primaire d'HLA-G fournit une copie complète (G1) de 1 200 pb, un fragment de 900 pb (G2) et un fragment de 600 pb (G3). Le transcrit G1 ne comprend pas l'exon 7 et correspond à la séquence décrite par ELLIS et al. (précité), c'est-à-dire qu'il code une protéine qui comprend une séquence signal, trois domaines externes, une région transmembranaire et une séquence cytoplasmique. L'ARNm G2 ne comprend pas l'exon 3, c'est-à-dire qu'il code une protéine dans laquelle les domaines α1 et α3 sont directement joints ; l'ARNm G3 ne contient ni l'exon 3, ni l'exon 4, c'est-à-dire qu'il code une protéine dans laquelle le domaine α1 et la séquence transmembranaire sont directement joints. L'épissage qui prévaut pour l'obtention de l'antigène HLA-G2 entraîne la jonction d'une adénine (A) (provenant du domaine codant α1) avec une séquence AC (issue du domaine codant α3), ce qui entraîne la création d'un codon AAC (asparagine) à la place du codon GAC (acide aspartique), rencontré au début de la séquence codant le domaine α3 dans HLA-G1. L'épissage généré pour l'obtention de HLA-G3 n'entraîne pas la formation d'un nouveau codon dans la zone d'épissage.

Les auteurs de cet article ont également analysé les différentes protéines exprimées : les 3 ARNm sont traduits en protéine dans la lignée cellulaire .221-G. Ils concluent sans le démontrer à un rôle fondamental de la molécule HLA-G dans la protection du foetus vis-à-vis d'une réponse immune maternelle (induction d'une tolérance immune).

Certains des inventeurs ont confirmé ce rôle : les molécules HLA-G, exprimées à la surface des trophoblastes protègent effectivement les cellules foetales de la lyse par les cellules *natural killer* (NK) maternelles (CAROSELLA E.D. et al., C.R. Acad. Sci., 1995, 318, 827-830; CAROSELLA E.D. et al, Trends Immunol. Today, 1996, 17, 9, 407-409).

En outre, certains des inventeurs ont montré l'existence d'autres formes épissées d'ARNm d'HLA-G : le transcrit HLA-G4, qui n'inclut pas l'exon 4 ; le transcrit HLA-G5, qui inclut l'intron 4, entre les exons 4 et 5, provoquant ainsi une modification du cadre de lecture, lors de la traduction de ce transcrit et en particulier l'apparition d'un codon stop, après l'acide aminé 21 de l'intron 4 ; le transcrit HLA-G6, possédant l'intron 4, mais ayant perdu l'exon 3 (KIRSZENBAUM M. et al., Proc. Natl. Acad Sci. USA, 1994, 91, 4209-4213 ; Demande Européenne EP 0 677 582 ; KIRSZENBAUM M. et al., Human Immunol., 1995, 43, 237-241 ; MOREAU P. et al., Human Immunol. 1995, 43, 231-236) ; et le transcrit HLA-G7 qui inclut l'intron 2, provoquant ainsi une modification du cadre de lecture, lors de la traduction de ce transcrit et l'apparition d'un codon stop après l'acide aminé 2 de l'intron 2 ; ils ont également montré que ces différents transcrits sont exprimés dans plusieurs types de cellules humaines foetales et adultes, notamment dans les lymphocytes T et B (KIRSZENBAUM M. et al., Human Immunol., 1995, précité ; MOREAU P. et al., Human Immunol. 1995, précité).

Il existe donc au moins 7 ARNms HLA-G différents qui codent potentiellement 7 isoformes d'HLA-G dont 4 membranaires (HLA-G1, G2, G3 et G4) et 3 solubles (HLA-G5, G6 et G7).

Des premières études ont montré que l'expression des molécules HLA-G à la surface de cellules cibles obtenues par transfection avec des vecteurs comprenant l'ADN génomique de HLA-G, générant potentiellement tous les transcrits alternatifs, permet de protéger lesdites cellules cibles de l'activité lytique des cellules NK de la couche déciduale de l'endomètre maternel (CHUMBLEY G. et al., Cell Immunol., 1994, 155, 312-322 ; DENIZ G. et al., J. Immunol., 1994, 152, 4255-4261).

Ces premières études ont été par la suite confirmées ; ainsi, aussi bien les isoformes liées à la membrane que les isoformes solubles sont immunotolérantes :
- elles inhibent la cytolyse médiée par les cellules NK et les CTL ;
- elles inhibent la réponse T alloproliférative. La littérature, dont celle émanant du groupe de M. CAROSELLA, décrit l'action inhibitrice d'HLA-G sur les cellules T (5-13) ;
- elles induisent une apoptose dans les cellules T et les cellules NK CD8⁺.

Ainsi la protéine HLA-G exerce sa fonction localement, aussi bien lorsqu'elle est exprimée à la surface des cellules que lorsqu'elle est sécrétée (action à distance) ; elle assure ainsi l'immunosurveillance de l'organisme (Teyssier E. et al., Nat. Immunol., 1995, 14, 262-270).

Ses propriétés d'immunotolérance ont également été démontrées *in vitro* dans de nombreux modèles de lignées tumorales transfectées par HLA-G (6, 23, 24). Les antigènes HLA-G jouent un rôle clé dans l'établissement et le maintien de l'immunotolérance en inhibant les fonctions des cellules immunocompétentes.

De tels effets inhibiteurs sont médiés par la liaison directe d'HLA-G à des récepteurs inhibiteurs spécifiques qui sont : ILT-2 (immunoglobulin-like transcript-2) (CD85j), exprimé par les cellules B, quelques cellules T, quelques cellules NK, les monocytes et les cellules dendritiques, ILT-4 (CD85d), exprimé par les cellules des lignées myéloïdes et KIR2DL4/p49 (CD158d) (Cantoni C. et *al.,* 1998 ; Rajagopalan S. et *al.,* 1999 ; Naji et al., 2007), exprimé par un sous-ensemble CD56^{bright} des cellules NK (1-4).

Ces propriétés sont partagées par l'ensemble des isoformes.

Ainsi HLA-G et notamment les isoformés solubles telles qu'HLA-G5:
- induisent l'apoptose des cellules T CD8+ et des cellules NK activées par liaison au récepteur CD8 et la stimulation de la voie Fas/Fas.
- exercent leurs effets inhibiteurs par un mécanisme de *feedback* parce qu'elles inhibent la réponse proliférative des cellules T CD4+ alloréactives qui la sécrètent.
- ont des propriétés immunosuppressives qu'elles exercent à travers leur interaction avec les récepteurs inhibiteurs différemment exprimés à la surface des différentes cellules immunitaires (cellules NK, cellules T, cellules B et cellules présentatrices des antigènes) (Carosella et al., Trends in Immunology, 2008, 29, 3, 125-132 ; Demande de Brevet FR 2 810 047 ; Naji et al., 2007).
- exercent également leur activité immunosuppressive par des effets médiés par des cytokines, telles que l'IL-10 et les interférons (Demande de Brevet FR 2810047).

Les propriétés tolérogéniques d'HLA-G ont des effets bénéfiques dans les désordres de la grossesse, de la transplantation et de l'auto-immunité et dans les maladies inflammatoires en limitant les réactions immunes, tandis qu'elles ont des effets délétères dans le cancer et après des infections virales en permettant l'échappement des cellules tumorales ou des cellules infectées par les virus.

Ainsi, il est aujourd'hui largement reconnu que l'expression de HLA-G par des cellules tumorales est un facteur négatif permettant à celles-ci d'inhiber la réponse antitumorale à travers l'interaction de HLA-G avec les récepteurs inhibiteurs de type ILT-2 exprimés par les cellules T et NK infiltrant la tumeur (voir le numéro spécial de la revue *Seminars in Cancer Biology* sur HLA-G et cancer (16)). Cette action de HLA-G conduit donc à la progression tumorale et le blocage de HLA-G est à ce titre proposé aujourd'hui comme nouvelle approche thérapeutique antitumorale. A titre d'exemple, on peut citer les travaux de l'équipe de M. Carosella sur le mélanome montrant le rôle de HLA-G dans la protection de cellules mélanomateuses contre l'action du système immunitaire (17-20). Cette observation est confirmée dans d'autres types de tumeurs telles que des lignées de gliome ou de carcinome rénal humain protégées d'une réponse cytotoxique allogénique par l'expression des molécules HLA-G1 et HLA-G5 (10,21,22). Les nombreuses revues sur le rôle de HLA-G en oncologie confirment ces observations (2,14,15,25-27).

Toutefois, l'ensemble de ces travaux concernent l'action de la molécule HLA-G exprimée dans des tumeurs solides.

De manière surprenante, les inventeurs ont maintenant montré que les formes solubles d'HLA-G ont une action antiproliférative sur les cellules B du système immunitaire. Par exemple, ils ont montré en particulier l'action inhibitrice des formes solubles d'HLA-G sur les fonctions de différenciation, de prolifération et de sécrétion d'anticorps des lymphocytes B. Ces résultats ont un impact particulièrement déterminant dans le cadre d'hémopathies malignes de la cellule B (lymphome, leucémie lymphoïde, myélome, syndrome de Burkitt, maladie de Hodgkin...).

La présente invention a en conséquence pour objet l'utilisation d'une forme soluble d'HLA-G pour la préparation d'un médicament pour le traitement ou la prévention des hémopathies malignes de la cellule B, c'est-à-dire des pathologies dans lesquelles on observe une prolifération anormale des cellules B du système immunitaire.

En d'autres termes, la présente invention concerne les formes d'HLA-G solubles pour utilisation comme médicament pour le traitement ou la prévention des hémopathies malignes de la cellule B.

Une telle utilisation de HLA-G en cancérologie, dans laquelle les cellules B sont tumorales, est particulièrement inattendue, puisqu'elle va à l'encontre du concept actuel du rôle de HLA-G comme mécanisme d'échappement tumoral à l'immunosurveillance (2,14,15).

Or, les inventeurs ont trouvé qu'HLA-G inhibe spécifiquement la prolifération des cellules tumorales du système immunitaire exprimant des récepteurs inhibiteurs d'HLA-G, c'est-à-dire principalement les cellules B. HLA-G inhibe également la prolifération, la différenciation en plasmocytes et la capacité à secréter des anticorps des cellules B anormalement activées.

### Définitions

### Hémopathies malignes de la cellule B

Les hémopathies malignes (ou cancers hématologiques) sont des cancers ou tumeurs liquides, c'est-à-dire des tumeurs dont les cellules circulent dans un liquide (sang ou lymphe), dans lesquelles on observe une prolifération anormale des cellules B. Parmi ces cancers liquides, on distingue les cancers du sang (leucémie), de la moelle osseuse (myélome, macroglobulinémie) ou des ganglions (lymphomes). Les hémopathies malignes de la cellule B incluent donc :
- la leucémie aiguë lymphoblastique de type B (LAL B), qui concerne les progéniteurs lymphoïdes (sang et moelle osseuse);
- la leucémie lymphoïde chronique (LLC) qui concerne les cellules B-1 CD5 (sang) ;
- la leucémie pré-B, qui concerne les cellules pré-B (sang et moelle osseuse) ;
- la maladie de Hodgkin (lymphome) qui concerne les cellules B des centres germinatifs ;
- les lymphomes non hodgkiniens, tels que le lymphome de Burkitt ou le lymphome folliculaire, qui concernent les cellules B mémoire matures de la périphérie ou le lymphome à cellule du manteau qui concerne les cellules B naïves au repos de la périphérie ;
- la macroglobulinémie de Waldenström qui concerne les cellules B secrétant des IgM (plasmocytes) et
- le myélome multiple (ou maladie de Kahler).

A titre d'exemple, les lymphomes non hodgkiniens sont des tumeurs malignes du système lymphatique ; il en existe de nombreuses formes qui évoluent de façon très différente les unes des autres.

Ces lymphomes se développent à partir des lymphocytes T ou B. Les tumeurs à cellules B représentent 75 % des cas dans les pays occidentaux alors que les tumeurs à cellules T sont plus courantes en Asie orientale.

L'incidence des lymphomes non hodgkiniens augmente dans le monde ; plus de 287 000 nouveaux cas surviennent chaque année, principalement dans les pays développés.

Les lymphomes non hodgkiniens se trouvent plus souvent dans les pays développés (52 % du nombre total de cas dans le monde) ou leur incidence a augmenté au cours des 20 dernières années, principalement en Amérique du Nord, Europe occidentale, Australie, Israël, Arabie Saoudite. Le lymphome est également une complication tumorale observée dans 5 à 10 % des cas de sida.

### Cellules B anormalement activées

On dit que les cellules B sont anormalement activées lorsqu'elles répondent à des auto-antigènes.

### Forme soluble d'HLA-G

Ladite forme soluble d'HLA-G est sélectionnée dans le groupe constitué par HLA-G5, HLA-G6 et HLA-G7, de préférence HLA-G5. Ces formes solubles sont bien connues de l'homme du métier.

L'utilisation d'HLA-G dans les cancers liquides constituent un traitement alternatif ou complémentaire, en association avec les traitements habituellement mis en oeuvre, tels que décrits par exemple dans Keating M. et al. (Hematology, 2003, 153-175); Dighiero G. et al. (The Lancet, 2008, 371, 1017-1029) ; ou Auer R. et al., (Br. J. Hematol., 2007, 139, 635-644).

Les formes solubles d'HLA-G, qui présentent un mécanisme d'action radicalement différent des autres produits anticancéreux habituellement mis en oeuvre, présentent ainsi, seules ou en association avec ces autres produits, un intérêt dans les cas (i) de mauvais taux de réponse avec les autres traitements, (ii) d'apparition de résistances aux autres traitements et (iii) lorsque les effets indésirables observés avec les autres traitements sont trop importants.

HLA-G5 et de manière plus générale, les formes solubles d'HLA-G, ont, dans les cancers liquides, une activité antiproliférative et limitent la progression tumorale.

Cette activité est opposée à celle antérieurement décrite, concernant les cancers solides dans lesquels on cherche à bloquer l'expression d'HLA-G, pour éliminer la tumeur solide.

Conformément à l'invention, la forme soluble d'HLA-G mise en oeuvre se présente :
- soit sous forme libre (ou monomérique), qui peut éventuellement former des dimères en solution,
- soit sous forme multimérique, notamment sous forme agrégée sur des billes, afin que la molécule d'HLA-G se présente sous forme de multimères, décrits comme étant la conformation fonctionnellement optimale de la molécule HLA-G. En effet, les dimères de HLA-G ont été décrits comme présentant une affinité considérablement augmentée pour les récepteurs de HLA-G comparativement aux monomères.

Conformément à l'invention, la prolifération anormale des cellules B est inhibée aussi bien par les formes solubles d' HLA-G purifiées et non agrégées sur des billes qu'avec les formes agrégées desdites formes solubles d'HLA-G.

La présente invention a également pour objet une composition pharmaceutique comprenant une forme soluble d'HLA-G et au moins un véhicule pharmaceutiquement acceptable pour utilisation comme médicament pour le traitement ou la prévention des hémopathies malignes de la cellule B.

Selon un mode de réalisation avantageux de ladite composition, ledit véhicule pharmaceutiquement acceptable est adapté à l'administration parentérale.

L'administration peut être par exemple intraveineuse, intramusculaire ou sous-cutanée.

Selon un mode de réalisation avantageux de ladite composition, ledit véhicule pharmaceutiquement acceptable est adapté à l'administration par inhalation.

Des solutions ou des suspensions utilisées pour l'application sous cutanée incluent typiquement l'un ou plusieurs des composés suivants : un diluant stérile, tel que l'eau, pour préparation injectable, une solution saline physiologique, isotonique et tamponnée, des huiles, des polyéthylène glycols, la glycérine, le polypropylène glycol ou d'autres solvants synthétiques ; des agents antibactériens tels que l'alcool benzylique ou les méthylparabènes ; des antioxydants tels que l'acide ascorbique ou le bisulfite de sodium ; des agents chélatants tels que l'acide éthylènediamine tétraacétique ; des tampons tels que des acétates, des citrates ou des phosphates ; et des agents pour l'ajustement de la tonicité tels que le chlorure de sodium ou le dextrose. Le pH peut être ajusté avec des acides ou des bases tels que l'acide chlorhydrique ou l'hydroxyde de sodium.

De telles préparations peuvent se présenter sous la forme d'ampoules, de seringues à jeter ou de flacons multidoses en verre ou en plastique.

Les compositions pharmaceutiques adaptées à l'injection incluent des solutions aqueuses stériles, des dispersions ou des poudres stériles pour une préparation extemporanée de solutions ou de dispersions injectables stériles.

Pour l'administration en intraveineuse, les véhicules préférés incluent les solutions salines physiologiques, l'eau bactériostatique, Cremophor ELTM (BASF, Parsippany, NJ) ou le tampon PBS. Dans tous les cas, la composition doit être stérile et fluide. Elle doit être stable dans les conditions de préparation et de stockage et doit comprendre des conservateurs contre l'action contaminante des microorganismes tels que les bactéries ou les champignons.

A titre d'exemple, le véhicule peut être un solvant ou un milieu de dispersion contenant par exemple de l'eau, de l'éthanol, un polyol (par exemple le glycérol, le propylène glycol ou un polyéthylène glycol liquide) et des mélanges de ces composés.

La fluidité correcte peut être maintenue par exemple en utilisant de la lécithine, ou en utilisant des agents surfactifs. La prévention de l'action des microorganismes peut être obtenue par l'administration de différents agents antibactériens et antifongiques, par exemple les parabènes, le chlorobutanol, le phénol, l'acide ascorbique et le thimérosal. Lesdites compositions peuvent également inclure des agents isotoniques, par exemple des sucres ou des polyalcools tels que le mannitol, le sorbitol ou le chlorure de sodium.

Les actions prolongées des compositions injectables peuvent être obtenues en ajoutant dans la formulation du monostéarate d'aluminium ou de la gélatine.

La présente invention a également pour objet des produits contenant une forme soluble d'HLA-G et un produit anticancéreux en tant que préparation combinée pour une utilisation simultanée, séparée ou séquentielle dans le traitement ou la prévention des cancers des hémopathies malignes de la cellule B.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

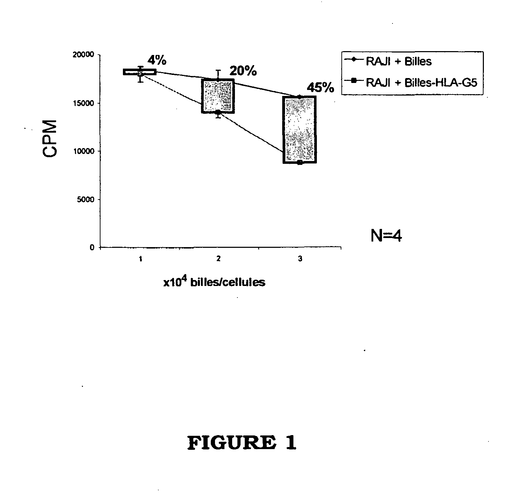

- la figure 1 illustre l'inhibition de la prolifération des cellules de la lignée tumorale B Raji (numéro d'accès ATCC : CCL-86).

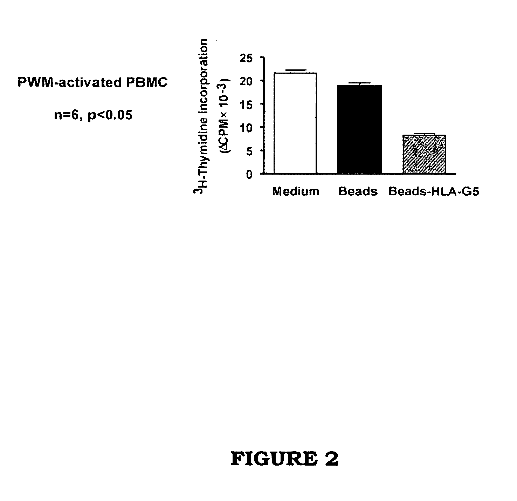

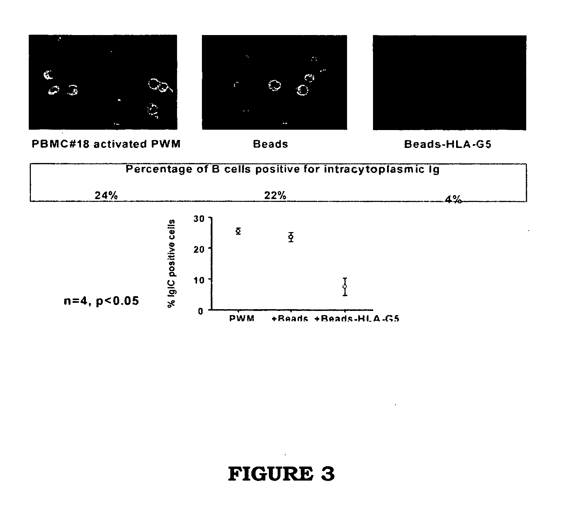

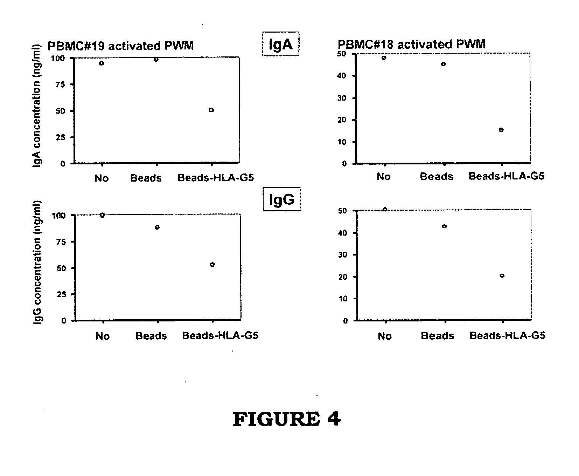

- les figures 2, 3 et 4 illustrent l'activité inhibitrice de HLA-G5 sur des cellules B normales stimulées par des agents mitogéniques ; figure 2 : action inhibitrice de HLA-G5 sur la prolifération des cellules B ; figure 3 : action inhibitrice de HLA-G5 sur la différenciation des cellules B en plasmocytes ; figure 4 : action inhibitrice de HLA-G5 sur la capacité des cellules B à sécréter des anticorps.

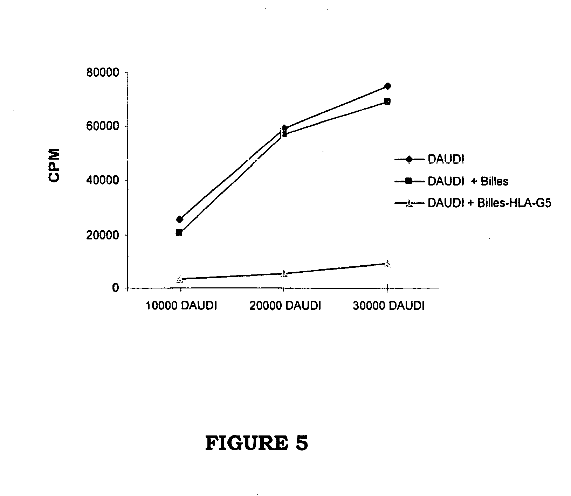

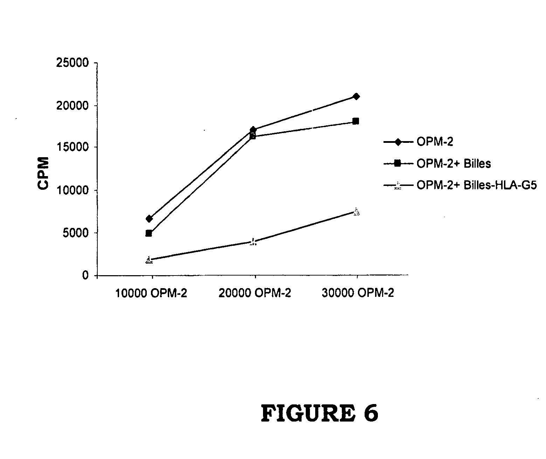

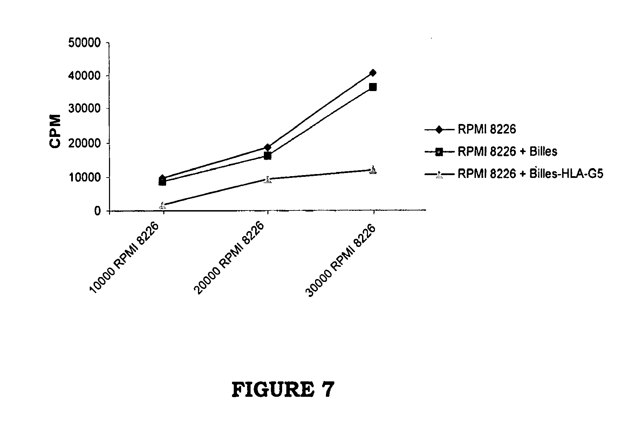

- les figures 5, 6 et 7 illustrent l'inhibition de la prolifération des cellules de différentes lignées ; figure 5 : lignée tumorale Daudi (numéro d'accès ATCC : CCL-213) ; figure 6 : lignée tumorale OPM-2 (numéro d'accès DSMZ : ACC 50) ; figure 7 : lignée tumorale RPMI 8226 (numéro d'accès ATCC : CCL-155).

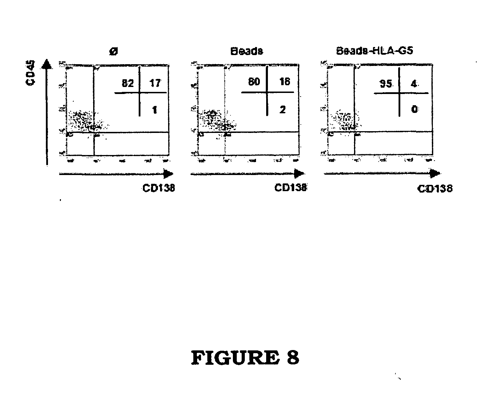

- la figure 8 illustre l'inhibition de la différenciation en cellules plasmocytaires malignes CD138⁺ des cellules CD138- de la moelle osseuse de patients atteints de myélome multiple ; les cellules ont été sensibilisées en présence (*Beads-HLA-G5*) ou en l'absence (∅ et *beads*) de HLA-G5. Seule une des 3 répétitions est représentée. Pour les cellules sensibilisées avec le milieu de culture sans billes (« ∅ ») : 17 cellules CD138- sur 100 se sont différenciées en cellules CD138⁺ ; pour les cellules sensibilisées avec le milieu de culture comprenant des microbilles seules (*« beads* ») : 18 cellules CD138⁻ sur 100 se sont différenciées en cellules CD138⁺ ; pour les cellules sensibilisées avec le milieu de culture comprenant des microbilles recouvertes de HLA-G5 (*Beads-HLA-G5*): 4 cellules CD138- sur 100 se sont différenciées en cellules CD138⁺.

Il doit être bien entendu, toutefois, que ces exemples sont donnés uniquement à titre d'illustration de l'objet de l'invention, dont ils ne constituent en aucune manière une limitation.

### EXEMPLE 1 : Matériels et méthodes

### - Cellules et cultures cellulaires

Deux lignées tumorales humaines de lymphomes de Burkitt obtenues à partir de patients atteints d'un syndrome de Burkitt (Raji, numéro d'accès ATCC : CCL-86 et Daudi, numéro d'accès ATCC : CCL-213) et deux lignées cellulaires de myélome (RPMI 8226, numéro d'accès ATCC : CCL-155 et OMP-2, numéro d'accès DSMZ : ACC 50) ont été obtenues.

Toutes les cellules sont cultivées sur milieu RPMI contenant 10 % de sérum de veau foetal, 2 mM de L-glutamine, de la fungizone et de la gentamicine.

Les cellules mononucléées du sang périphérique (PBMC) sont isolées à partir de donneurs volontaires sains (Etablissement français du sang, Hôpital St Louis, Paris, France). Les PBMC de sang total héparinisé de donneurs sains sont obtenues par centrifugation en gradient de densité sur Ficoll-histopaque 1077 (Sigma).

Des fractions cellulaires CD138⁻ ont été fournies par Pasteur-Cerba (Cergy, France). Ces fractions cellulaires correspondent à des cellules mononucléées de la moelle osseuse (BM), isolées à partir de prélèvements effectués chez des patients atteints de myélome multiple, et dont les cellules plasmocytaires CD138⁺ en ont été exclues en utilisant des microbilles magnétiques anti-CD138 humain (Miltenyi Biotech). Le consentement éclairé a été obtenu de tous les patients selon la Déclaration d'Helsinki, et l'étude a été approuvée par le comité d'éthique.

### - Anticorps

- W6/32 : IgG2a spécifique des molécules HLA de classe I associées à la β2 microglobuline β2 m) (Sigma-Aldrich).
- 5A6G7 : IgG1 anti-HLA-G5 et G6 (décrits dans Le Rond et al., Eur. J. Immunol., 2004, 34, 649-660 ; Menier et al., Blood, 2004).

### - Production d'HLA-G5

La protéine HLA-G5 et sa séquence nucléique sont décrites dans la Demande de Brevet EP 0 677 582. La production d'une forme soluble d'une isoforme d'HLA-G chez un baculovirus est décrite en détail à l'exemple 1 de la Demande EP 1 189 627.

Brièvement, la protéine HLA-G5 recombinante est produite dans des cellules d'insecte SF9, cultivées sur milieu TNMFH contenant 5 % de sérum de veau foetal (Invitrogen), infectées avec un baculovirus contenant la séquence codant pour HLA-G5 (baculovirus HLA-G5) ou infectées par le baculovirus HLA-G5 ainsi qu'avec un baculovirus codant pour la β2m humaine (Appligene) et cultivées pendant 5 jours à 27°C en présence de 5 % de CO₂.

La protéine HLA-G5 apyrogène est purifiée à partir du surnageant de culture des cellules SF9 infectées par chromatographie d'immunoaffinité avec des anticorps monoclonaux W6/32 (Sigma-Aldrich).

### - Production d'HLA-G5 recombinante adsorbée sur des microbilles

Brièvement, les microbilles magnétiques sont des particules mono-dispersées d'un diamètre de 300 nm et sont recouvertes d'IgG de chèvre anti-souris liées de manière covalente à leur surface (Bio-Adembeads goat anti-mouse, Ademtech).

Ces microbilles sont incubées une nuit à 4°C avec des anticorps monoclonaux 5A6G7 (Exbio), spécifiques d'HLA-G5.

Après lavage, les microbilles recouvertes d'anticorps 5A6G7 sont incubées avec le milieu contenant HLA-G5 à 4°C pendant 2h.

La capture d'HLA-G est vérifiée par analyse en Western blot, dans les conditions décrites dans Le Rond et al., Eur. J. Immunol., 2004, précité.

### - Tests de prolifération cellulaire

Les lignées cellulaires tumorales sont ensemencées dans trois puits à 10⁴ cellules/100 µl de milieu contenant des quantités croissantes soit de microbilles recouvertes d'HLA-G5, soit de microbilles seules (contrôle négatif).

Après 24h, les cultures sont pulsées avec de la ³H-thymidine (1 µCi/puits, Amersham, Biosciences).

Les cellules sont récupérées 18h plus tard et l'incorporation de la thymidine dans l'ADN est quantifiée sur un compteur β (Wallac 1450, Pharmacia).

Les cellules mononucléées du sang périphérique (PBMC) (10⁵ cellules/puits) sont activées par le mitogène pokeweed (2 µg/ml) en l'absence ou en présence des billes HLA-G5 ou des billes seules (2.10³ billes/cellule).

Après 5 jours, les cultures sont pulsées avec de la ³H-thymidine (1 µCi/puits, Amersham, Biosciences). Les cellules sont récoltées 18 heures plus tard, et l'incorporation de la thymidine dans l'ADN est quantifiée sur un compteur P (Wallac 1450, Pharmacia).

### - Analyse du cycle cellulaire

Les cellules Raji sont traitées soit avec les billes HLA-G5 soit avec les billes seules (5.10⁴ billes/cellule). Après 24h, les cellules Raji sont fixées dans de l'éthanol à 70 % (v/v) dans un tampon PBS et incubées une nuit à 4°C. Les cellules lavées sont incubées dans un tampon PBS contenant 40 µg/ml d'iodure de propidium (Sigma) et 100 µg/ml de DNase sans RNase A dans de la glace, au moins 10 min.

Les paramètres du cycle cellulaire sont obtenus à l'aide d'un cytomètre de flux LSR^{™} et le logiciel Cell Quest^{™} (Becton Dickinson).

La distribution du cycle cellulaire est déterminée par analyse automatique mettant en oeuvre le logiciel ModFit LT^{™} avec AutoDebris^{™} et AutoAggregates^{™}. Le pourcentage de cellules dans chaque phase du cycle cellulaire (G0/G1, S et G2) est calculé comme décrit dans Menier C. et al. (Leukemia, 2008, 22, 578-584).

### - Coloration par immunofluorescence des immunoglobulines intracytoplasmiques

Les PBMC (10⁶ cellules/ml) sont activées par le mitogène pokeweed (2 µg/ml) en l'absence ou en présence des billes HLA-G5 ou des billes seules (2.10³ billes/cellule).

Après 5 jours, les cellules sont récoltées à partir des cultures par la technique des cytospins (lames superfrost/plus^{®} (Merck, Strasbourg)) et Cytospin 3^{®} (Shandon).

Pour la coloration, les cellules sont fixées dans de l'éthanol et incubées 30 min avec des anticorps de chèvre anti-IgG, anti-IgA et anti-IgM humaines marqués au FITC (fluorescéine 5-isothiocyanate) ou des anticorps de contrôle marqués au FITC.

Les noyaux sont marqués en rouge avec de l'iodure de propidium.

Les lames sont analysées à l'aide d'un microscope à fluorescence (Biorad MRC 1024). Le pourcentage de plasmocytes positifs pour Ig intracyto-plasmique est établi par comptage des cellules avec un cytoplasme fluorescent.

### - ELISA

Les PBMC (10⁶ cellules/ml) sont activées par le mitogène pokeweed (2 µg/ml) en l'absence ou en présence des billes HLA-G5 ou des billes seules (2.10³ billes/cellule). Les IgA et IgG humaines sécrétées dans les surnageants de culture de PBMC sont mesurées à l'aide des kits de quantification IgG ELISA et IgA ELISA (Bethyl, Montgomery, TX), selon les instructions du fabricant.

### - Tests de différenciation cellulaire

Les fractions cellulaires CD138⁻ obtenues à partir de prélèvements de moelle osseuse effectués chez des patients atteints de myélome multiple ont été sensibilisées *in vitro,* pendant 18 à 24 h, avec du milieu de culture contenant soit des microbilles recouvertes de HLA-G5, soit des microbilles seules, ou du milieu de culture ne contenant pas de microbilles. Les cellules ont ensuite été récupérées (sans les microbilles) et cultivées pendant 21 jours.

Après les 3 semaines de culture, l'expression de CD 138 à la surface des cellules parmi la population de cellules CD45⁺ a été déterminée par cytométrie en flux.

### - Cytométrie en flux

Les anticorps utilisés pour les analyses de cytométrie en flux ont été conjugués au FITC, PE (Phycoérythrine), DPE (dinitrophényle) ou PC5 (Phycoérythriné-cyanine 5) (Beckman Coulter et BD Pharmingen). Brièvement, les cellules ont été incubées 30 min à 4°C dans 20% de sérum humain et ensuite marquées avec les anticorps. Le contrôle isotype a été systématiquement utilisé pour évaluer et compenser le signal non spécifique. Les cellules ont été analysées sur un cytomètre en flux EPIC XL4 en utilisant le logiciel Expo32 (Beckman Coulter).

### - Analyse statistique

Toutes les données sont représentatives des expériences réalisées au moins trois fois. La significativité a été évaluée par le test t non apparié (*unpaired t test*), considérant que p<0,05 est significatif.

### EXEMPLE 2: Inhibition de la prolifération de cellules tumorales B (lignée tumorale B Raji).

La lignée cellulaire tumorale Raji décrite à l'exemple 1 a été traitée par des billes seules ou des billes recouvertes de la forme soluble HLA-G5. Après 24 heures, la prolifération des cellules tumorales B Raji a été analysée par incorporation de thymidine tritiée. Le pourcentage d'inhibition de la prolifération est représenté à la figure 1 et correspond à la moyenne obtenue sur quatre expériences.

Ces résultats sont illustrés dans la Figure 1 avec la lignée tumorale B Raji, dérivée d'un patient atteint d'un syndrome de Burkitt, dont la prolifération est significativement réduite après traitement par la forme soluble HLA-G5 et ceci de façon dose-dépendante. Cette inhibition de la prolifération des cellules tumorales B Raji par HLA-G5 passe par un arrêt en phase G1 du cycle cellulaire (Tableau I).

**TABLEAU I**

| | Go/G1 | S | G2 |
|---|---|---|---|
| Raji | 60% | 30% | 10% |
| Raji+Billes | 60% | 30% | 10% |
| Raji+Billes-HLA-G5 | 100% | | |

La distribution au cours du cycle cellulaire a été définie par un marquage à l'iodure de propidium des cellules Raji après 24h d'un traitement avec des billes-HLA-G5 ou des billes seules à raison de 50.000 billes / cellule ce qui correspond à 50ng/ml de HLA-G5. Les résultats sont représentés en pourcentage de cellules dans chaque phase du cycle.

### EXEMPLE 3: Inhibition de la prolifération de cellules tumorales B (lignée DAUDI, lignée OPM-2 et lignée RPMI 8226s

Des résultats similaires à ceux de l'exemple 2 ont été obtenus avec une autre lignée tumorale B de Burkitt, la lignée Daudi ainsi qu'avec des lignées issues d'un autre type de lymphoprolifération que sont les myélomes.

Les lignées cellulaires Daudi, OPM-2 et RPMI 8226 décrites à l'exemple 1 ont été traitées par des billes seules ou des billes recouvertes de la forme soluble HLA-G5 ou n'ont pas été traitées. Après 24 heures, la prolifération de ces cellules tumorales B a été analysée par incorporation de thymidine tritiée. Le nombre de cellules tumorales introduites dans le test varie de 10000 à 30000 cellules par puits. Le nombre de billes est fixe et est de 50000 billes par cellule.

Les billes recouvertes de la protéine HLA-G5 inhibent dans tous les cas la prolifération de ces cellules tumorales B (figures 5, 6 et 7).

Cette expérience est représentative de trois expériences indépendantes.

### EXEMPLE 4: Activité inhibitrice de HLA-G5 sur des cellules B normales stimulées par des agents mitogéniques (pockeweed mitogen ou Pansorbine).

### - Action inhibitrice de HLA-G5 sur la prolifération des cellules B (figure 2)

Des cellules mononucléées de sang périphérique (PBMC) isolées à partir d'un prélèvement sanguin d'individus sains ont été stimulées par l'agent mitogénique pockeweed mitogen (PWM) en présence (Beads-HLA-G5) ou en l'absence (Beads) de billes recouvertes de la forme soluble HLA-G5. Après 5 jours, la prolifération des cellules B stimulées a été analysée par incorporation de thymidine tritiée. Ces résultats représentent la moyenne obtenue dans 6 expériences indépendantes. L'inhibition de prolifération liée au traitement par HLA-G5 est statistiquement significative. La figure 2 illustre ces résultats.

### - Action inhibitrice de HLA-G5 sur la différenciation des cellules B en plasmocytes (figure 3)

Des cellules mononucléées de sang périphérique (PBMC) isolées à partir d'un prélèvement sanguin d'individus sains ont été stimulées par l'agent mitogénique pockeweed mitogen (PWM) en présence (Beads-HLA-G5) ou en l'absence (Beads) de billes recouvertes de la forme soluble HLA-G5. Après 5 jours, le pourcentage de cellules B différenciées en plasmocytes présentant des immunoglobulines intracytoplasmiques (IgIC) a été défini par immunofluorescence. L'inhibition de différenciation liée au traitement par HLA-G5 est statistiquement significative. La figure 3 illustre ces résultats.

### - Action inhibitrice de HLA-G5 sur la capacité des cellules B à sécréter des anticorps (figure 4)

Des cellules mononucléées de sang périphérique (PBMC) isolées à partir d'un prélèvement sanguin d'individus sains ont été stimulées par l'agent mitogénique pockeweed mitogen (PWM) en présence (Beads-HLA-G5) ou en absence (Beads) de billes recouvertes de la forme soluble HLA-G5. Après 5 jours, le taux d'immunoglobulines IgA et IgG dans les surnageants de culture a été mesuré par technique immunoenzymatique. L'inhibition de sécrétion d'anticorps liée au traitement par HLA-G5 est statistiquement significative. La figure 4 illustre ces résultats.

### EXEMPLE 5 : Inhibition ex vivo, par HLA-G5, de la différenciation de cellules CD138⁻ de la moelle osseuse de patients atteints de myélome multiple en cellules plasmatiques malignes CD138⁺.

Des fractions cellulaires CD138- obtenues à partir de moelle osseuse de patients atteints de myélome multiple ont été sensibilisées pendant 18h à 24h avec du milieu de culture contenant soit des microbilles recouvertes de HLA-G5, soit des microbilles seules, ou du milieu de culture ne contenant pas de microbilles. Après 3 semaines de culture sans microbilles, la différenciation des cellules CD138- en cellules CD138⁺ a été analysée par cytométrie en flux.

Les résultats sont représentés à la figure 8. Il ressort de cette figure qu'HLA-G5 inhibe, à hauteur de 68% ((17x4)/100), la capacité des cellules progéniteurs CD138⁻ à se différencier en cellules cancéreuses CD138⁺. En revanche, en l'absence de HLA-G5 (∅ et *beads),* un nombre significativement plus grand de cellules progéniteurs CD138- se différencient en cellules plasmocytaires malignes CD138⁺ (respectivement 17/100 et 18/100).

### Bibliographie

1. Carosella ED et al., Blood, 2008, 111, 4862-4870.
2. Carosella ED et al., Trends Immunol., 2008, 29, 125-132.
3. Colonna M et al., J Exp Med., 1997, 186, 1809-1818.
4. Cosman D et al., Immunity, 1997, 7, 273-282.
5. Rouas-Freiss N et al., Proc Natl Acad Sci U S A. , 1997, 94, 5249-5254.
6. Riteau B et al., J Immunol., 2001,166, 5018-5026.
7. Lila N et al., Proc Natl Acad Sci U S A., 2001, 98, 12150-12155.
8. Ristich V et al., Eur J Immunol., 2005, 35, 1133-1142.
9. Liang S et al., Eur J Immunol., 2002, 32, 2418-2426.
10. Wiendl H et al., Jlmmunol., 2002, 168, 4772-4780.
11. Wiendl H et al., Brain, 2003, 126, 176-185.
12. Le Friec G et al., Hum Immunol., 2003, 64, 752-761.
13. Naji A et al., Hum Immunol., 2007, 68, 233-239.
14. Rouas-Freiss N et al., Semin Cancer Biol., 2007, 17, 413-421.
15. Urosevic M et al., Cancer Res., 2008, 68, 627-630.
16. Carosella ED et al., Semin Cancer Biol., 2007, 17, 411-412.
17. Paul P et al., Proc Natl Acad Sci U S A., 1998, 95, 4510-4515.
18. Adrian Cabestre F et al., J Reprod Immunol., 1999, 43, 183-193.
19. Adrian Cabestre F et al., Semin Cancer Biol., 1999, 9, 27-36.
20. Rouas-Freiss N et al., Int J Cancer, 2005, 117, 114-122.
21. Bukur J et al., Cancer Res., 2003, 63, 4107-4111.
22. Seliger B et al., Semin Cancer Biol., 2007, 17, 444-450.
23. Menier C et al., Int J Cancer, 2002, 100, 63-70.
24. Caumartin J et al., Embo J., 2007, 26, 1423-1433.
25. Rouas-Freiss N et al., Cancer Res. , 2005, 65, 10139-10144.
26. Rouas-Freiss N et al., Semin Cancer Biol., 2003, 13, 325-336.
27. Seliger B et al., Trends Immunol., 2003, 24, 82-87.
28. Geraghty et al., Proc. Natl. Acad Sci. USA, 1987, 84, 9145-9149.
29. Carosella E.D. et al., C.R. Acad Sci., 1995, 318, 827-830.
30. Carosella E.D. et al, Trends Immunol. Today, 1996, 17, 9, 407-409.
31. Kovats et al., Science, 1990, 248, 220-223.
32. Crisa et al., J Exp Med., 1997, 186, 289-298.
33. Cirulli et al., Diabetes, 2006, 55, 1214-1222.
34. Ishitani et al., Proc. Natl. Acad Sci. USA, 1992, 89, 3947-3951.
35. Ellis et al., J. Immunol., 1990, 144, 731-735.
36. Kirszenbaum M. et al., Proc. Natl. Acad Sci. USA, 1994, 91, 4209-4213.
37. Kirszenbaum M. et al., Human Immunol., 1995, 43, 237-241.
38. Moreau P. et al., Human Immunol. 1995, 43, 231-236.
39. Chumbley G. et al., Cell Immunol., 1994, 155, 312-322.
40. Deniz G. et al., J Immunol., 1994, 152, 4255-4261.
41. Teyssier E. et al., Nat. Immunol., 1995, 14, 262-270.
42. Carosella et al., Adv Immunol., 2003, 81, 199-252.
43. Cantoni C. et al., Eur. J. Immunol., 1998, 28, 6, 1980-90.
44. Rajagopalan S. et al., J. Exp. Med., 1999, 189, 7, 1093-100.
45. Keating M. et al., Hematology, 2003, 153-175.
46. Dighiero G. et al., The Lancet, 2008, 371, 1017-1029.
47. Auer R. et al., (Br. J. Hematol., 2007, 139, 635-644.
48. Le Rond et al., Eur. J. Immunol., 2004, 34, 649-660.
49. Naji et al., Blood, 2007, 110, 12, 3936-3948.
50. Menier C. et al., Blood, 2004, 104, 10, 3153-3160.

## Revendications

1. Forme soluble d'HLA-G pour utilisation comme médicament pour le traitement ou la prévention des hémopathies malignes de la cellule B.

2. Composition pharmaceutique comprenant une forme soluble d'HLA-G et au moins un véhicule pharmaceutiquement acceptable pour utilisation comme médicament pour le traitement ou la prévention des hémopathies malignes de la cellule B.

3. Forme soluble d'HLA-G pour utilisation selon la revendication 1, **caractérisée en ce que** ladite forme soluble d'HLA-G est sélectionnée dans le groupe constitué par HLA-G5, HLA-G6 et HLA-G7, de préférence HLA-G5.

4. Forme soluble d'HLA-G pour utilisation selon la revendication 1 ou la revendication 3, **caractérisée en ce que** ladite forme soluble est sous forme libre ou monomérique.

5. Forme soluble d'HLA-G pour utilisation selon la revendication 1 ou la revendication 3, **caractérisée en ce que** ladite forme soluble est sous forme multimérique.

6. Composition pour utilisation selon la revendication 2, **caractérisée en ce que** ladite forme soluble d'HLA-G est sélectionnée dans le groupe constitué par HLA-G5, HLA-G6 et HLA-G7, de préférence HLA-G5.

7. Composition pour utilisation selon la revendication 2 ou la revendication 6, **caractérisée en ce que** ladite forme soluble est sous forme libre ou monomérique.

8. Composition pour utilisation selon la revendication 2 ou la revendication 6, **caractérisée en ce que** ladite forme soluble est sous forme multimérique.

9. Composition pour utilisation selon l'une quelconque des revendications 2 et 6 à 8, **caractérisée en ce que** ledit véhicule pharmaceutiquement acceptable est adapté à l'administration parentérale.

10. Composition pour utilisation selon l'une quelconque des revendications 2 et 6 à 8, **caractérisée en ce que** ledit véhicule pharmaceutiquement acceptable est adapté à l'administration par inhalation.

11. Produits contenant une forme soluble d'HLA-G et un produit anticancéreux en tant que préparation combinée pour une utilisation simultanée, séparée ou séquentielle dans le traitement ou la prévention des cancers des hémopathies malignes de la cellule B.

12. Forme soluble d'HLA-G pour utilisation selon l'une quelconque des revendications 1 et 3 à 5, **caractérisée en ce que** lesdites hémopathies malignes de la cellule B sont choisies dans le groupe constitué par la leucémie aigüe lymphoblastique de type B, la leucémie lymphoïde chronique, la leucémie pré-B, la maladie de Hodgkin, les lymphomes non hodgkiniens, la macroglobulinémie de Waldenström et le myélome multiple.

13. Composition pour utilisation selon l'une quelconque des revendications 2 et 6 à 10, **caractérisée en ce que** lesdites hémopathies malignes de la cellule B sont choisies dans le groupe constitué par la leucémie aigüe lymphoblastique de type B, la leucémie lymphoïde chronique, la leucémie pré-B, la maladie de Hodgkin, les lymphomes non hodgkiniens, la macroglobulinémie de Waldenstrôm et le myélome multiple.

14. Produits pour utilisation selon la revendication 11, **caractérisés en ce que** lesdites hémopathies malignes de la cellule B sont choisies dans le groupe constitué par la leucémie aiguë lymphoblastique de type B, la leucémie lymphoïde chronique, la leucémie pré-B, la maladie de Hodgkin, les lymphomes non hodgkiniens, la macroglobulinémie de Waldenström et le myélome multiple.

## Patentansprüche

1. Lösliche Form von HLA-G zur Verwendung als Medikament für die Behandlung oder die Prävention maligner Hämopathien der B-Zelle.

2. Pharmazeutische Zusammensetzung, umfassend eine lösliche Form von HLA-G und wenigstens ein pharmazeutisch annehmbares Vehikel zur Verwendung als Medikament für die Behandlung oder die Prävention maligner Hämopathien der B-Zelle.

3. Lösliche Form von HLA-G zur Verwendung gemäß Anspruch 1, **dadurch gekennzeichnet, dass** die lösliche Form von HLA-G ausgewählt ist aus der Gruppe, bestehend aus HLA-G5, HLA-G6 und HLA-G7, vorzugsweise HLA-G5 ist.

4. Lösliche Form von HLA-G zur Verwendung gemäß Anspruch 1 oder Anspruch 3, **dadurch gekennzeichnet, dass** die lösliche Form in freier oder monomerer Form ist.

5. Lösliche Form von HLA-G zur Verwendung gemäß Anspruch 1 oder Anspruch 3, **dadurch gekennzeichnet, dass** die lösliche Form in multimerer Form ist.

6. Zusammensetzung zur Verwendung gemäß Anspruch 2, **dadurch gekennzeichnet, dass** die lösliche Form von HLA-G aus der Gruppe, bestehend aus HLA-G5, HLA-G6 und HLA-G7 ausgewählt ist, vorzugsweise HLA-G5 ist.

7. Zusammensetzung zur Verwendung gemäß Anspruch 2 oder Anspruch 6, **dadurch gekennzeichnet, dass** die lösliche Form in freier Form oder monomerer Form ist.

8. Zusammensetzung zur Verwendung gemäß Anspruch 2 oder Anspruch 6, **dadurch gekennzeichnet, dass** die lösliche Form in multimerer Form ist.

9. Zusammensetzung zur Verwendung gemäß einem der Ansprüche 2 und 6 bis 8, **dadurch gekennzeichnet, dass** das pharmazeutisch annehmbare Vehikel zur parenteralen Verabreichung angepasst ist.

10. Zusammensetzung zur Verwendung gemäß einem der Ansprüche 2 und 6 bis 8, **dadurch gekennzeichnet, dass** das pharmazeutisch annehmbare Vehikel zur Verabreichung durch Inhalation angepasst ist.

11. Produkte, die eine lösliche Form von HLA-G und ein Antikrebsprodukt enthalten, als Kombinationspräparat für eine gleichzeitige, getrennte oder sequentielle Verwendung bei der Behandlung oder Prävention von Krebs der malignen Hämopathien der B-Zelle.

12. Lösliche Form von HLA-G zur Verwendung gemäß einem der Ansprüche 1 und 3 bis 5, **dadurch gekennzeichnet, dass** die malignen Hämopathien der B-Zelle ausgewählt sind aus der Gruppe, bestehend aus akuter lymphoblastischer Leukämie des Typs B, chronischer lymphatischer Leukämie, Pre-B-Leukämie, Hodgkin-Erkrankung, Nicht-Hodgkin-Lymphomen, Waldenström-Makroglobulinämie und multiplem Myelom.

13. Zusammensetzung zur Verwendung gemäß einem der Ansprüche 2 und 6 bis 10, **dadurch gekennzeichnet, dass** die malignen Hämopathien der B-Zelle ausgewählt sind aus der Gruppe, bestehend aus akuter lymphoblastischer Leukämie des Typs B, chronischer lymphatischer Leukämie, Pre-B-Leukämie, Hodgkin-Erkrankung, Nicht-Hodgkin-Lymphomen, Waldenström-Makroglobulinämie und multiplem Myelom.

14. Produkte zur Verwendung gemäß Anspruch 11, **dadurch gekennzeichnet, dass** die malignen Hämopathien der B-Zelle ausgewählt sind aus der Gruppe, bestehend aus akuter lymphoblastischer Leukämie des Typs B, chronischer lymphatischer Leukämie, Pre-B-Leukämie, Hodgkin-Erkrankung, Nicht-Hodgkin-Lymphomen, Waldenström-Makroglobulinämie und multiplem Myelom.

## Claims

1. A soluble form of HLA-G for use as a medicament for the treatment or prevention of B-cell malignant hemopathies.

2. A pharmaceutical composition comprising a soluble form of HLA-G and at least one pharmaceutically acceptable vehicle for use as a medicament for the treatment or prevention of B-cell malignant hemopathies.

3. The soluble form of HLA-G for use as claimed in claim 1, **characterized in that** said soluble form of HLA-G is selected from the group comprising HLA-G5, HLA-G6 and HLA-G7, preferably HLA-G5.

4. The soluble form of HLA-G for use as claimed in claim 1 or claim 3, **characterized in that** said soluble form is in the free or monomeric form.

5. The soluble form of HLA-G for use as claimed in claim 1 or claim 3, **characterized in that** said soluble form is in the multimeric form.

6. The composition for use as claimed in claim 2, **characterized in that** said soluble form of HLA-G is selected from the group comprising HLA-G5, HLA-G6 and HLA-G7, preferably HLA-G5.

7. The composition for use as claimed in claim 2 or claim 6, **characterized in that** said soluble form is in the free or monomeric form.

8. The composition for use as claimed in claim 2 or claim 6, **characterized in that** said soluble form is in the multimeric form.

9. The composition for use as claimed in any one of claims 2 and 6 to 8, **characterized in that** said pharmaceutically acceptable vehicle is suitable for parenteral administration.

10. The composition for use as claimed in any one of claims 2 and 6 to 8, **characterized in that** said pharmaceutically acceptable vehicle is suitable for administration by inhalation.

11. Products containing a soluble form of HLA-G and an anticancer product as combined preparation for simultaneous, separate or sequential use in the treatment or prevention of cancers of B-cell malignant hemopathies.

12. The soluble form of HLA-G for use as claimed in any one of claims 1 and 3 to 5, **characterized in that** said B-cell malignant hemopathies are selected from the group consisting of acute lymphoblastic leukemia of type B, chronic lymphocytic leukemia, pre-B leukemia, Hodgkin's disease, non-Hodgkin lymphomas, Waldenstrom macroglobulinemia and multiple myeloma.

13. The composition for use as claimed in any one of claims 2 and 6 to 10, **characterized in that** said B-cell malignant hemopathies are selected from the group consisting of acute lymphoblastic leukemia of type B, chronic lymphocytic leukemia, pre-B leukemia, Hodgkin's disease, non-Hodgkin lymphomas, Waldenstrom macroglobulinemia and multiple myeloma.

14. The products for use as claimed in claim 11, **characterized in that** said B-cell malignant hemopathies are selected from the group consisting of acute lymphoblastic leukemia of type B, chronic lymphocytic leukemia, pre-B leukemia, Hodgkin's disease, non-Hodgkin lymphomas, Waldenstrom macroglobulinemia and multiple myeloma.
